(19) **Europäisches Patentamt / European Patent Office / Office européen des brevets**

(11) **EP 4 186 524 A1**

(12) # EUROPEAN PATENT APPLICATION
published in accordance with Art. 153(4) EPC

(43) Date of publication:
**31.05.2023 Bulletin 2023/22**

(21) Application number: **21846233.1**

(22) Date of filing: **20.07.2021**

(51) International Patent Classification (IPC):
**A61K 41/00** (2020.01)    **A61F 9/007** (2006.01)
**A61K 31/525** (2006.01)    **A61K 31/661** (2006.01)
**A61K 31/7084** (2006.01)    **A61K 41/17** (2020.01)
**A61P 27/10** (2006.01)

(52) Cooperative Patent Classification (CPC):
**A61F 9/007; A61K 31/525; A61K 31/661;**
**A61K 31/7084; A61K 41/00; A61K 41/17;**
**A61P 27/10**

(86) International application number:
**PCT/JP2021/027154**

(87) International publication number:
**WO 2022/019304 (27.01.2022 Gazette 2022/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.07.2020   JP 2020124445**

(71) Applicants:
- **Santen Pharmaceutical Co., Ltd.**
  **Kita-ku**
  **Osaka-shi**
  **Osaka 530-8552 (JP)**
- **Fibertech Co., Ltd**
  **Sakura-shi, Chiba, 285-0074 (JP)**

(72) Inventors:
- **OHASHI, Koji**
  **Ikoma-shi, Nara 630-0101 (JP)**
- **KOYAMA, Yukie**
  **Ikoma-shi, Nara 630-0101 (JP)**
- **TANAKA, Yasuhisa**
  **Ikoma-shi, Nara 630-0101 (JP)**
- **OKABE, Komei**
  **Ikoma-shi, Nara 630-0101 (JP)**
- **OGAWA, Toshihiro**
  **Ikoma-shi, Nara 630-0101 (JP)**
- **KAMIKATANO, Mitsuru**
  **Funabashi-shi, Chiba 273-0864 (JP)**

(74) Representative: **Mewburn Ellis LLP**
**Aurora Building**
**Counterslip**
**Bristol BS1 6BX (GB)**

(54) **PREVENTION AND/OR TREATMENT OF EYE DISEASE BY COLLAGEN FIBER CROSSLINKING**

(57)    The present invention provides an improvement in the strength of the scleral by collagen fiber crosslinking via crosslinking treatment of the sclera at the equatorial region of the eye and/or at the posterior pole such as the fundus of the eye. Also provided is the prevention and/or treatment of eye disease by the same. Provided are: a photosensitizer used in a crosslinking treatment for the sclera at the equatorial region of the eye and/or at the posterior pole of the eye; a kit containing the same; and a method for emitting light for the crosslinking treatment.

EP 4 186 524 A1

**Description**

TECHNICAL FIELD

The present invention relates to prevention and/or treatment of eye diseases by crosslinking of collagen fibers via crosslinking treatment.

BACKGROUND ART

Corneal crosslinking treatment may be performed these days in Europe and the United States to treat keratoconus which is a disease resulting in protrusion of the center of the cornea of the eye in a conical shape. The corneal crosslinking treatment is a method of treatment to prevent progression of keratoconus by irradiation of ultraviolet light with a long wavelength (at around 365 nm) to the cornea for 30 minutes while administering a solution of riboflavin (vitamin B2) as a photosensitizer in the cornea, thereby causing crosslinking formation of collagen fibers in the parenchyma of the cornea and enhancing the strength of collagen fibers.

Myopia is a state in which the axial length of the eyeball (the length from the cornea peak to the central fovea) and the adjustment of the refractive power of the lens are unbalanced and an image is focused short of the retina, which causes blurred vision out of focus. Axial myopia or excessive myopia refers to a condition wherein the axial length of the eyeball extends anteroposteriorly, causing elongation of the retina or choroid backward and thereby letting the eyeball under load. Furthermore, excessive myopia that indicates various abnormalities on the fundus of the eye such as posterior staphyloma caused by the load refers to as pathological myopia. Excessive myopia including many of pathological myopia is the leading cause of blindness, but no definitive therapy curing degeneration of the eyeball has been unknown.

The sclera constitutes the fibrous layer of the eyeball with the cornea and comprises collagen fibers like the cornea, and researches have been performed to enhance the strength of collagen fibers and suppress elongation of axial length of the eyeball by crosslinking treatment on the sclera as symptomatic therapy for pathological myopia.

Non patent literature 1 discloses that collagen crosslinking treatment with a solution of riboflavin and UVA (ultraviolet A) increased the strength of sclera sections of human and porcine.

Non patent literature 2 discloses that collagen crosslinking treatment with a solution of riboflavin and UVA (ultraviolet A) in the rabbit sclera *in vivo* maintained the strength of the sclera over a long term after the treatment.

Non patent literature 3 discloses the effect of irradiation time and safety during collagen crosslinking treatment with a solution of riboflavin and UVA (ultraviolet A) in the rabbit sclera *in vivo.*

A certain level of procedures of corneal crosslinking treatment with a solution of riboflavin and ultraviolet light has been established, whereas crosslinking treatment to the sclera has not been sufficiently established in terms of the light intensity for irradiation and the irradiation time so that collagen fibers are efficiently subjected to crosslinking without substantive adverse effects on living bodies. In addition, conventional crosslinking treatment is limited to the anterior surface of the eyeball from the ocular surface to the equatorial region of the eye where a solution of riboflavin can be administered dropwise.

CITATION LIST

NON PATENT LITERATURE

Non Patent Literature 1 J Cataract Refract Surg 2004, Vol. 30, 689-695
Non Patent Literature 2 Acta Ophthalmol. 2009, Vol. 87, 193-198
Non Patent Literature 3 J Cataract Refract Surg 2013, Vol. 39, 1184-1189

SUMMARY OF INVENTION

PROBLEMS TO BE SOLVED BY THE INVENTION

One of interesting problems is to provide crosslinking treatment on the sclera of the equatorial region of the eye and/or the posterior pole of the eye such as the fundus of the eye, thereby enhancing the strength of the sclera via crosslinking of collagen fibers without substantive adverse effects on living bodies, and thus resulting in prevention and/or treatment of an eye disease.

MEANS OF SOLVING THE PROBLEMS

The present inventors have found and achieved the invention including a photosensitizer for use in crosslinking treatment on the sclera of the equatorial region of the eye and/or posterior pole of the eye and a kit comprising the same and a method of emitting light for crosslinking treatment.

Specifically, some embodiments are provided as follows.

(1) A photosensitizer composition for use in prevention and/or treatment of myopia, comprising a photoexcitable compound, wherein the prevention and/or treatment comprises crosslinking treatment in the sclera of the equatorial region of the eye or the posterior pole of the eye.

(2) The composition of item (1), wherein the composition is used to be administered to the subconjunctiva or the capsule of Tenon of the eye.

(3) The composition of item (1) or (2), wherein the crosslinking treatment comprises emitting toward the sclera light comprising light having a wavelength absorbed by the photoexcitable compound.

(4) The composition of any one of items (1) to (3), wherein the light in the crosslinking treatment comprises light having a wavelength ranging from 300 to 550 nm.

(5) The composition of any one of items (1) to (4), wherein the crosslinking treatment comprises emitting toward the sclera light having an illuminance ranging from 1 to 30 mW/cm$^2$.

(6) The composition of any one of items (1) to (5), wherein the crosslinking treatment comprises emitting light toward the sclera for 1 to 10 minutes.

(7) The composition of any one of items (1) to (6), wherein the crosslinking treatment comprises emitting light in the presence of the atmosphere.

(8) The composition of any one of items (1) to (7), wherein the crosslinking treatment comprises emitting light comprising light having a wavelength of 365 nm at an illuminance of 10 mW/cm$^2$ for 5 minutes.

(9) The composition of any one of items (1) to (8), for use as an injection.

(10) The composition of any one of items (1) to (9), wherein the photoexcitable compound is comprised in a concentration ranging from 0.001 to 1%(w/v).

(11) The composition of any one of items (1) to (10), wherein the photoexcitable compound is riboflavin, flavin mononucleotide, flavin adenine dinucleotide, or a salt or a hydrate thereof.

(12) The composition of any one of items (1) to (11), wherein the myopia is at least one myopia selected from the group consisting of axial myopia, excessive myopia, and pathological myopia.

(13) The composition of any one of items (1) to (12), wherein the myopia is associated with posterior staphyloma.

(14) A kit comprising the photosensitizer composition of any one of items (1) to (13) and a probe for emitting light comprising light having a wavelength absorbed by the photoexcitable compound comprised in the photosensitizer composition.

(15) The kit of item (14), wherein the probe is used to be arranged along the sclera at the equatorial region of the eye or the posterior pole of the eye.

(16) The kit of item (14) or (15), wherein the probe comprises an emission surface for emitting light, to which a member comprising at least one pore is attached.

(17) The kit of any one of items (14) to (16), wherein the light comprises light having a wavelength ranging from 300 to 550 nm.

(18) The kit of any one of items (14) to (17), wherein the light is emitted with an illuminance ranging from 1 to 30 mW/cm$^2$ .

(19) The kit of any one of items (14) to (18), wherein the probe is used to emit light for 1 to 10 minutes.

(20) The kit of any one of items (14) to (19), wherein the photosensitizer composition is enclosed in a unit-dose container.

(21) The kit of any one of items (14) to (20), further comprising a light source device connectable to the probe.

(22) The kit of any one of items (14) to (21), further comprising a cautionary statement or a package insert indicating that the photosensitizer composition and the probe may be used or are to be used for the prevention and/or treatment of myopia,

(23) A kit comprising a probe and a light source device, wherein the probe comprises:

a linear light-guiding body that guides light from the light source device;
a planar emission body that is connected to the leading end part of the linear light-guiding body and emits light;
a reflective surface that is provided on a portion of the planar emission body and reflects the light incident on the planar emission body; and
an emission surface that is provided to oppose the reflective surface and allows the light reflected on the reflective surface to be emitted in a planar manner.

(24) The kit of item (23), wherein the probe is used to be arranged along the sclera at the equatorial region of the eye or the posterior pole of the eye.

(25) The kit of item (23) or (24), wherein the probe comprises an emission surface for emitting light, to which a member comprising at least one pore is attached.

(26) The kit of any one of items (23) to (25), wherein the light comprises light having a wavelength ranging from 300 to 550 nm.

(27) The kit of any one of items (23) to (26), wherein the light is emitted from the probe with an illuminance ranging from 1 to 30 mW/cm$^2$.

(28) The kit of any one of items (23) to (27), wherein the probe is used to emit light for 1 to 10 minutes.

(29) The kit of any one of items (23) to (28), further comprising the photosensitizer composition of any one of items (1) to (13).

(30) The kit of any one of items (23) to (29), wherein the photosensitizer composition is enclosed in a unit-dose container.

(31) The kit of any one of items (23) to (30), further comprising a cautionary statement or a package insert indicating that the probe and the light source device may be used or are to be used for the prevention and/or treatment of myopia.

(32) A method for preventing and/or treating myopia, comprising the following steps:

(1) administering a photosensitizer composition comprising a photoexcitable compound to the subconjunctiva or the capsule of Tenon of the eye, and
(2) emitting toward the sclera light comprising light having a wavelength absorbed by the photoexcitable compound, thereby resulting in crosslinking of a collagen fiber contained in the sclera.

(33) The method of item (32), wherein the light comprises light having a wavelength ranging from 300 to 550 nm.

(34) The method of item (32) or (33), wherein the light is emitted with an illuminance ranging from 1 to 30 mW/cm$^2$ for 1 to 10 minutes.

(35) The method of any one of items (32) to (34), comprising in the step (2) emitting light comprising light having a wavelength of 365 nm with an illuminance of 10 mW/cm$^2$ for 5 minutes.

(36) The method of any one of items (32) to (35), wherein the administration step is carried out by injection.

(37) The method of any one of items (32) to (36), wherein the photoexcitable compound is comprised in the composition in a concentration ranging from 0.001 to 1%(w/v).

(38) The method of any one of items (32) to (37), wherein the photoexcitable compound is riboflavin, flavin mononucleotide, flavin adenine dinucleotide, or a salt or a hydrate thereof.

(39) The method of any one of items (32) to (38), comprising increasing the ratio of crosslinking of collagen fibers in the sclera by emitting the light in the presence of the atmosphere.

(40) The method of any one of items (32) to (39), further comprising, before or after the step (1), arranging at least a part of a probe for emitting the light along the sclera at the equatorial region of the eye or the posterior pole of the eye.

(41) The method of any one of items (32) to (40), wherein the myopia is at least one myopia selected from the group consisting of axial myopia, excessive myopia, and pathological myopia.

(42) The method of any one of items (32) to (40), wherein the myopia is associated with posterior staphyloma.

(43) A method for crosslinking a collagen fiber, comprising administering a photosensitizer composition comprising a photoexcitable compound to the subconjunctiva or the capsule of Tenon of the eye, wherein the composition is involved in crosslinking of a collagen fiber in the sclera of the equatorial region of the eye or the posterior pole of the eye under the irradiation of light comprising light having a wavelength absorbed by the photoexcitable compound.

(44) The method of item (43), comprising increasing the ratio of crosslinking of collagen fibers in the sclera by irradiation of the light in the presence of the atmosphere.

(45) The method of item (43) or (44), comprising irradiating light comprising light having a wavelength of 365 nm with an illuminance of 10 mW/cm$^2$ for 5 minutes.

(46) The method of any one of items (43) to (45), wherein the photoexcitable compound is riboflavin, flavin mononucleotide, flavin adenine dinucleotide, or a salt or a hydrate thereof.

(47) A method of emitting light, comprising emitting toward the sclera light comprising light having a wavelength ranging from 300 to 550 nm at an illuminance ranging from 1 to 30 mW/cm$^2$ for 1 to 10 minutes.

(48) The method of item (47), comprising forming a crosslinked collagen fiber in the sclera by using a photosensitizer composition comprising a photoexcitable compound that absorbs the light.

(49) The method of item (47) or (48), comprising increasing the ratio of crosslinking of collagen fibers in the sclera by emitting the light in the presence of the atmosphere.

(50) The method of any one of items (47) to (49), wherein the sclera is the sclera at the equatorial region of the eye or the posterior pole of the eye.

(51) The method of any one of items (47) to (50), wherein the photoexcitable compound is riboflavin, flavin mononucleotide, flavin adenine dinucleotide, or a salt or a hydrate thereof.

(52) Use of a photoexcitable compound in the manufacture of a photosensitizer composition for use in prevention and/or treatment of myopia, wherein the prevention and/or treatment comprises crosslinking treatment in the sclera of the equatorial region of the eye or the posterior pole of the eye.

(53) The use of item (52), wherein the composition is used for administration to the subconjunctiva or the capsule of Tenon of the eye.

(54) The use of item (52) or (53), wherein the crosslinking treatment comprises emitting toward the sclera light comprising light having a wavelength absorbed by the photoexcitable compound.

(55) The use of any one of items (52) to (54), wherein the light in the crosslinking treatment comprises light having a wavelength ranging from 300 to 550 nm.

(56) The use of any one of items (52) to (55), wherein the crosslinking treatment comprises emitting toward the sclera light having an illuminance ranging from 1 to 30 mW/cm$^2$.

(57) The use of any one of items (52) to (56), wherein the crosslinking treatment comprises emitting light toward the sclera for 1 to 10 minutes.

(58) The use of any one of items (52) to (57), wherein the crosslinking treatment comprises emitting light in the presence of the atmosphere.

(59) The use of any one of items (52) to (58), wherein the crosslinking treatment comprises emitting light comprising light having a wavelength of 365 nm with an illuminance of 10 mW/cm$^2$ for 5 minutes.

(60) The use of any one of items (52) to (59), wherein the composition is for use as an injection.

(61) The use of any one of items (52) to (60), wherein the photoexcitable compound is comprised in a concentration ranging from 0.001 to 1%(w/v).

(62) The use of any one of items (52) to (61), wherein the photoexcitable compound is riboflavin, flavin mononucleotide, flavin adenine dinucleotide, or a salt or a hydrate thereof.

(63) The use of any one of items (52) to (62), wherein the myopia is at least one myopia selected from the group consisting of axial myopia, excessive myopia, and pathological myopia.

(64) The use of any one of items (52) to (62), wherein the myopia is associated with posterior staphyloma.

(65) A photoexcitable compound for use in prevention and/or treatment of myopia, wherein the prevention and/or treatment comprises crosslinking treatment of the sclera in the equatorial region of the eye or the posterior pole of the eye.

(66) The compound for use of item (65), wherein the photoexcitable compound is for use in administration to the subconjunctiva or the capsule of Tenon of the eye.

(67) The compound for use of item (65) or (66), wherein the crosslinking treatment comprises emitting toward the sclera light comprising light having a wavelength absorbed by the photoexcitable compound.

(68) The compound for use of any one of items (65) to (67), wherein the light in the crosslinking treatment comprises light having a wavelength ranging from 300 to 550 nm.

(69) The compound for use of any one of items (65) to (68), wherein the crosslinking treatment comprises emitting toward the sclera light having an illuminance ranging from 1 to 30 mW/cm$^2$.

(70) The compound for use of any one of items (65) to (69), wherein the crosslinking treatment comprises emitting light toward the sclera for 1 to 10 minutes.

(71) The compound for use of any one of items (65) to (70), wherein the crosslinking treatment comprises emitting light in the presence of the atmosphere.

(72) The compound for use of any one of items (65) to (71), wherein the crosslinking treatment comprises emitting light comprising light having a wavelength of 365 nm with an illuminance of 10 mW/cm$^2$ for 5 minutes.

(73) The compound for use of any one of items (65) to (72), wherein the compound is formulated into a composition for an injection.

(74) The compound for use of any one of items (65) to (73), wherein the photoexcitable compound is comprised in a composition in a concentration ranging from 0.001 to 1%(w/v).

(75) The compound for use of any one of items (65) to (74), wherein the photoexcitable compound is riboflavin, flavin mononucleotide, flavin adenine dinucleotide, or a salt or a hydrate thereof.

(76) The compound for use of any one of items (65) to (75), wherein the myopia is at least one myopia selected from the group consisting of axial myopia, excessive myopia, and pathological myopia.

(77) The compound for use of any one of items (65) to (75), wherein the myopia is associated with posterior staphyloma.

Any two or more of optionally selected elements in each of the above items may be combined with each other.

EFFECT OF THE INVENTION

The present invention allows for crosslinking treatment on the sclera of the equatorial region of the eye and/or the posterior pole of the eye such as the fundus of the eye, thereby enhancing the strength of the sclera via crosslinking of collagen fibers without substantive adverse effects on living bodies, and thus resulting in prevention and/or treatment of an eye disease.

DESCRIPTION OF EMBODIMENTS

The present invention is explained in detail as below.

The term "crosslinking treatment" as used herein refers to treatment of irradiating light having a certain wavelength with letting a photosensitizer permeated into a living body site, thereby resulting in crosslinking of collagen fibers at the irradiated site, so as to enhance the strength (stiffness) of collagen fibers. The term "crosslinking treatment (therapy)" includes any treatment/therapy of symptoms (e.g., alleviation, amelioration, remission, regression, or inhibition, prevention or delay of progression) performed by the crosslinking treatment and any prevention thereof (e.g., prevention or delay of development). In one embodiment of the crosslinking treatment, collagen fibers within the range of irradiation may be selectively subjected to crosslinking by adjusting the amount of a photosensitizer and the condition of irradiation light without substantive adverse effects on living bodies.

The term "living body site" as used herein may be any living tissues in need of crosslinking treatment and is not limited thereto, but includes narrow regions such as the region between the eyeball and the inside of the eyelid and the region of the eye constricted by pathology, specifically sclera or cornea. One embodiment of living body sites is the cornea and the sclera of the anterior eye segment ranging from the eye surface to the equatorial region of the eye. Another embodiment of living body sites is the sclera of the equatorial region of the eye and the back part of the eye ranging from the equatorial region of the eye to the posterior pole of the eye. A preferable one is the sclera of the equatorial region of the eye and/or the posterior pole of the eye. The term "corneal crosslinking treatment" is used when the living body site subjected to the crosslinking treatment is cornea, whereas the term "scleral crosslinking treatment" is used for sclera.

The term "a photosensitizer" (also referred to as "a photosensitizer composition" as used herein) as used herein refers to a formulation comprising a compound that is subjected to excitation by absorption of light (also referred to as a "photoexcitable compound" as used herein). The term "photoexcitable compound" as used herein includes riboflavin, flavin mononucleotide (FMN) (also referred to as "riboflavin-5'-phosphate" hereinafter), flavin adenine dinucleotide (FAD), and a salt or a hydrate thereof.

Herein, a photoexcitable compound that is comprised in a photosensitizer may be a single compound or a mixture of any two or more compounds, and is preferably a single compound. One embodiment of the "photoexcitable compound" is riboflavin-5'-phosphate or a salt or a hydrate thereof.

The salt, as used herein, may be any pharmaceutically acceptable salts but is not limited thereto, and includes salts with alkali metals such as sodium and potassium, and salts with alkali earth metals such as magnessium and calcium.

The content of a photoexcitable compound comprised in a photosensitizer herein may be adjusted. When the content of a photoexcitable compound is low, the effect of the crosslinking treatment is not sufficient. Whereas, when the content of a photoexcitable compound is high, the effect of the crosslinking treatment is not sufficient within a prescribed time period due to increase of necessary light intensity. For example, the content of a photoexcitable compound ranges, preferably from 0.0001 to 10%(w/v), more preferably from 0.001 to 1%(w/v), still preferably from 0.002 to 0.5%(w/v), still more preferably from 0.02 to 0.5%(w/v), particularly preferably from 0.02 to 0.2%(w/v), from 0.1 to 0.5%(w/v), or from 0.2 to 0.5%(w/v). More specifically, the content is particularly preferably 0.1%(w/v), 0.15%(w/v), 0.2%(w/v), 0.25%(w/v), 0.3%(w/v), 0.4%(w/v), or 0.5%(w/v).

The term "%(w/v)" as used herein refers to mass (g) of a target ingredient contained in 100 mL of a photosensitizer. For example, when a salt of riboflavin is contained therein, the value is the content of the salt of riboflavin. For example, when riboflavin or a salt thereof is contained therein in the form of a hydrate or a solvate, the value is the content of the hydrate or the solvate of riboflavin or a salt thereof. Herein, the same is applied unless otherwise specified.

The photosensitizer as used herein may further include an additive that is commonly used in the art, if necessary. The additive includes, specifically, tonicity agents, buffering agents, thickening agents, surfactants, stabilizing agents, antioxidants, preservatives, and pH adjusters. Each of these agents may be added to a photosensitizer alone or as a mixture of any two or more of them in an appropriate amount.

When a buffering agent is comprised in the photosensitizer herein, any buffering agents that are commonly available as an additive for medicine may be used. The buffering agent includes, for example, phosphoric acid or a salt thereof, boric acid or a salt thereof, and carbonic acid or a salt thereof, and may also include a hydrate or a solvate thereof.

Phosphoric acid or a salt thereof includes, for example, phosphoric acid, trisodium phosphate, sodium dihydrogen phosphate, sodium hydrogen phosphate (disodium hydrogen phosphate), tripotassium phosphate, potassium dihydrogen phosphate, and dipotassium hydrogen phosphate, and may also be a hydrate thereof.

Boric acid or a salt thereof includes, for example, boric acid, sodium borate (borax), and potassium borate, and may also be a hydrate thereof.

Carbonic acid or a salt thereof includes, for example, sodium carbonate and sodium hydrogen carbonate, and may also be a hydrate thereof.

When a buffering agent is comprised in the photosensitizer herein, the buffering agent is preferably phosphoric acid or phosphate, more preferably sodium dihydrogen phosphate or sodium hydrogen phosphate. Any two or more buffering agents may be combined.

When a buffering agent is comprised in the photosensitizer herein, the content of the buffering agent may be optionally adjusted depending on the buffering agent as used herein, but it ranges preferably from 0.001 to 10%(w/v), more preferably from 0.01 to 5%(w/v), still preferably from 0.1 to 2%(w/v), and particularly preferably from 0.1 to 1%(w/v).

When a tonicity agent is comprised in the photosensitizer herein, any tonicity agents that are commonly available as an additive for medicine may be used. The tonicity agent includes, for example, ionic tonicity agents and non-ionic tonicity agents. It is preferably ionic tonicity agents. Any two or more tonicity agents may be combined.

Ionic tonicity agents include, for example, sodium chloride, potassium chloride, calcium chloride, and magnesium chloride, and may also be a hydrate thereof. A preferable one is sodium chloride.

Non-ionic tonicity agents include, for example, glycerin, propylene glycol, polyethylene glycol, sorbitol, mannitol, trehalose, maltose, sucrose, and xylitol, and may also be a hydrate thereof.

When a tonicity agent is comprised in the photosensitizer herein, the content of the tonicity agent may be optionally adjusted depending on the tonicity agent as used herein, but it ranges preferably from 0.001 to 10%(w/v), more preferably from 0.01 to 5%(w/v), still preferably from 0.1 to 3%(w/v), and particularly preferably from 0.5 to 2%(w/v).

When a thickening agent is comprised in the photosensitizer herein, any thickening agents that are commonly available as an additive for medicine may be used. The thickening agent includes, for example, cellulose polymers such as hydroxyethylcellulose, hydroxypropyl methylcellulose, and sodium carboxymethyl cellulose; polyvinylpyrrolidone; carboxyvinyl polymer; polyols such as polyethylene glycol; and mucopolysaccharides such as sodium hyaluronate, and may also be a hydrate thereof.

When a thickening agent is comprised in the photosensitizer herein, the content of the thickening agent may be optionally adjusted depending on the thickening agent as used herein, but it ranges preferably from 0.001 to 5%(w/v), more preferably from 0.01 to 3%(w/v), and still preferably from 0.1 to 2%(w/v).

When a surfactant is comprised in the photosensitizer herein, any surfactants that are commonly available as an additive for medicine may be used. The surfactant includes, for example, cationic surfactants such as alkylamine salt and fatty acid triethanolamine ester salts; anionic surfactants such as alkyl benzene sulfonate and lecithin; and non-ionic surfactants such as polysorbate 80, polyoxyl 40 stearate, polyoxyethylene 40 hydrogenated castor oil, and polyoxyl 35 castor oil, and may also be a hydrate thereof.

When a surfactant is comprised in the photosensitizer herein, the content of the surfactant may be optionally adjusted depending on the surfactant as used herein, but it ranges preferably from 0.01 to 1%(w/v), more preferably from 0.05 to 0.5%(w/v), and still preferably from 0.05 to 0.2%(w/v).

When a stabilizing agent is comprised in the photosensitizer herein, any stabilizing agents that are commonly available as an additive for medicine may be used. The stabilizing agent includes, for example, edetic acid or a salt thereof, and may also be a hydrate thereof.

When a stabilizing agent is comprised in the photosensitizer herein, the content of the stabilizing agent may be optionally adjusted depending on the stabilizing agent as used herein, but it ranges preferably from 0.001 to 5%(w/v), more preferably from 0.01 to 3%(w/v), and still preferably from 0.1 to 2%(w/v).

When an antioxidant is comprised in the photosensitizer herein, any antioxidants that are commonly available as an additive for medicine may be used. The antioxidant includes, for example, ascorbic acid, tocopherol, dibutylhydroxytoluene, and sodium sulfite, and may be a hydrate thereof.

When an antioxidant is comprised in the photosensitizer herein, the content of the antioxidant may be optionally adjusted depending on the antioxidant as used herein, but it ranges preferably from 0.001 to 5%(w/v), more preferably from 0.01 to 3%(w/v), and still preferably from 0.1 to 2%(w/v).

When a preservative is comprised in the photosensitizer herein, any preservatives that are commonly available as an additive for medicine may be used. The preservative includes, for example, invert soaps such as benzalkonium chloride and benzethonium chloride; parabens such as methyl parahydroxybenzoate and propyl parahydroxybenzoate; alcohols such as chlorobutanol; and chlorhexidine hydrochloride, and may be a hydrate thereof.

When a preservative is comprised in the photosensitizer herein, the content of the preservative may be optionally adjusted depending on the preservative as used herein, but it ranges preferably from 0.0001 to 1%(w/v), more preferably from 0.001 to 0.5%(w/v), and still preferably from 0.001 to 0.1%(w/v).

When a pH adjuster is comprised in the photosensitizer herein, any pH adjusters that are commonly available as an additive for medicine may be used. The pH adjuster includes, for example, acids or bases. The acid includes, for example, hydrochloric acid, phosphoric acid, citric acid, and acetic acid, and the base includes, for example, sodium hydroxide, potassium hydroxide, sodium carbonate, and sodium hydrogen carbonate.

As used herein, pH of the photosensitizer may be any values acceptable for medicine, and it ranges, for example, from 4.0 to 8.5 or from 4.0 to 8.0, preferably from 6.0 to 8.0, more preferably from 6.5 to 7.5, particularly preferably from 6.7 to 7.3, and for example, includes 6.7, 6.8, 6.9, 7.0, 7.1, 7.2, and 7.3.

The osmotic pressure ratio of the photosensitizer as used herein may be any values acceptable for medicine, and it ranges, for example, from 0.5 to 2.0, preferably from 0.7 to 1.6, more preferably from 0.8 to 1.4, and still preferably from 0.9 to 1.2.

All of constituents of the photosensitizer as used herein may be dissolved, or the photosensitizer may be partly suspended. Preferably, the phososenstizer is in liquid form with all of the constituents being dissolved. A solvent or dispersion media as used herein is preferably water, and an aqueous solution is the most preferable.

The photosensitizer as used herein may be administered parenterally to a patient, and topical administration to the eye is more preferable. The topical administration to the eye as used herein includes, for example, administration with eye drops, administration in conjunctival sac, intravitreal administration, subconjunctival administration, and administration to the capsule of Tenon. One embodiment of the living body sites that are subjected to crosslinking treatment is preferably the sclera in the equatorial region of the eye and/or the posterior pole of the eye. In order to let a photosensitizer efficiently permeated into these living body sites, the topical administration to the eye is preferably subconjunctival administration or administration to the capsule of Tenon.

The photosensitizer as used herein may be optionally formulated with a pharmaceutically acceptable additive into a dosage form suitable for administration. A preferable dosage form is a dosage form suitable for parenteral administration and includes, for example, eye drops, injections, ophthalmic ointments, intercalating agents, and transdermal formulations. One embodiment of the living body sites that are subjected to crosslinking treatment is preferably the sclera in the equatorial region of the eye and/or the posterior pole of the eye. In order to let a photosensitizer efficiently permeated into these living body sites, the dosage form suitable for administration is preferably injections.

Doses and the frequency of administration of the photosensitizer as used herein may be, but not limited thereto, any doses and frequencies that allow for administration of the amount to be permeated into living body sites. For example, for crosslinking treatment in the sclera in the anterior eye segment ranging from the surface of the eye to the equatorial region of the eye, it is preferable to place drops of the photosensitizer in the eye directly or adjacent to the sclera site that is subjected to the crosslinking treatment during the treatment at a timing such as one time every 30 seconds, one time every 60 seconds, and one time every 90 seconds with 1 or 2 drops for one time. One drop herein ranges typically from about 0.01 to about 0.1 mL, preferably from about 0.015 to about 0.07 mL, and more preferably from about 0.02 to about 0.05 mL. In another embodiment, the living body site that is subjected to crosslinking treatment is preferably the sclera in the equatorial region of the eye and/or the posterior pole of the eye, and in this embodiment, a prescribed dose of the photosensitizer may be administered to the subconjunctiva or the capsule of Tenon only once or twice during the treatment. The prescribed dose for this embodiment may be optionally adjusted depending on the size of the eyeball of a subject, but for example in administration to human, it ranges typically from 0.1 to 3 mL, preferably from 0.2 to 2.5 mL, preferably from 0.5 to 2.5 mL, and more preferably from 1 to 2 mL or from 1.5 to 2.5 mL.

The photosensitizer as used herein may be contained in any containers without restriction in their materials, and the container may be, for example, glass containers; or resin containers made of polyethylene (PE), polypropylene (PP), polyethylene terephthalate (PET), polybutylene terephthalate (PBT), polypropylene-polyethylene copolymers, polyvinyl chloride, acrylic resins, polystyrene, or polycyclic olefin copolymers. The container may be either a multi-dose container available for multiple use or a unit-dose container for non-reusable single use, or a pre-filled syringe. The container is preferably a unit-dose container.

The photosensitizer as used herein may be prepared by commonly available methods. For example, ingredients are dissolved or suspended in distilled water, and the resultant is adjusted within prescribed ranges of osmotic pressure and pH, followed by filtration and sterilization, to give a photosensitizer.

The crosslinking treatment as used herein may be carried out with a probe that emits light toward a subject. The "probe" is an apparatus, device, or instrument for medical use, comprising a light-guiding body that guides light from a light source device to let light enter into the probe, and an emission body that is connected to the leading end part of the light-guiding body and emits the light, and may be used for living body sites to result in crosslinking of collagen fibers.

Emitting light as used herein refers to shooting light outward, and may be used as a synonym, for example, of injection, radiation, and irradiation of light.

The light-guiding body as used herein refers to a member for guiding light from a light source device to an emission body. The material of the light-guiding body is, for example, an optical fiber or an optical fiber bundle and may be coated with an exterior pipe. The material of the exterior pipe is, for example, a metal such as stainless steel. The shape of the light-guiding body includes, but is not limited thereto and for example, linear (including tubular) and planar. The linear shape is preferable so as to allow a probe to be used at a narrow space or strictured site of living bodies.

The emission body as used herein refers to a member that is connected to the leading end part of a light-guiding body and is configured to emit light incident from the light-guiding body. The material of the emission body is, for example, a transparent material such as acrylic resins. The shape of the emission body is not limited thereto, but is preferably planar so as to emit light with a desired range at one emission. It is preferable that the emission body does not have a corner at its leading end part so as to be used for insertion at a narrow space or strictured site of living bodies. The shape is more preferably oval, elongated round, ellipse, rounded rectangle, round, or planar having a part of any of these shapes. The shape of the emission body is more preferably discoidal or ellipse-plate shaped, and at least a part of the body may be either planar or curved and may have a concavity or convexity.

The size of the emission body as used herein is not limited thereto, but is preferably so small as to be used at a narrow space or strictured site of living bodies as well. The long side of the emission body has, for example, the length of preferably 30 mm or less, and more preferably 20 mm or less. When the emission body is discoidal or comprises a part of the discoidal shape, the diameter of the discoidal part is preferably 20 mm or less, more preferably 10 mm or less. The thickness of the emission body is up to preferably 10 mm or less, more preferably 5 mm or less.

The emission body as used herein comprises an emission surface. The light emitted from the emission body is emitted through the emission surface. The emission surface may be either planar or curved. The emission surface may be used in contact with living body sites, but preferably, the emission surface may be without contact with living body sites and the atmosphere is preferably retained between the emission surface and living body sites. For example, when the emission surface is planar, a plate-like member comprising at least one concave or penetrated pore, referred to as a "plate" hereinafter, may be further attached to the emission surface so as to allow for the probe to retain the atmosphere with the capacity of the pore. Preferably, the pore is a pore that penetrates the plate. The atmosphere existing between the emission surface and living body sites may allow for efficient crosslinking of collagen fibers. The shape, number, or size of the pore is not limited, and may be optionally adjusted to retain a necessary volume of the atmosphere. For example, the shape of the pore is cylindrical or elliptic cylindrical, and the diameter or the length of the major axis of the pore is 0.5 to 2 mm, preferably 1.5 mm or less. The material of the plate is preferably the same as the material of the emission body, for example, acrylic resins. In terms of the size of the plate, it is preferable that one side of the plate has the same area as the emission surface, and the thickness of the plate is preferably thin so as to be used at a narrow space or strictured site of living bodies. For example, it is preferably 2 mm or less, and more preferably around 1 mm.

The emission body as used herein may comprise a reflective surface that reflects light incident on the emission body to a desired direction, and the reflective surface may be arranged so as to oppose the emission surface, and thereby resulting in emitting from the emission surface light reflected from the reflective surface.

When the emission body is formed by a transparent material, at least a part of the emission body (for example, the reflective surface) may comprise a reflective coating because a part of light incident may not be emitted in a desired directon due to permeation and scattering, and light reflected from the reflective coating may be emitted toward a desired direction. The reflective coating may be made of, for example, metal such as aluminum and silver, and may be formed by vapor deposition of metal.

The probe as used herein may comprise a member in addition to the light-guiding body and the emission body, and the member includes, for example, a gripper for a user of the probe to grasp the probe, a cable for connecting the probe to the light source device, and a connection for connecting the light-guiding body to the emission body or connecting the light-guiding body to the cable. The base end section of the light-guiding body of the probe may be connected to the light source device via the gripper, the cable, and the connection.

The probe as used herein may be washed and sterilized to be reused after use, but is preferably non-reusable in terms of infection prevention.

The light source device as used herein is a device comprising one or more light sources, the device which is to emit light from the light source and to provide light with a probe connected to the device. The light source includes, but is not limited thereto, halogen lamps, xenon lamps, heavy hydrogen lamps, mercury lamps, and LED light sources.

The eye disease as used herein that is subjected to the treatment and/or prevention by crosslinking treatment includes, for example, eye diseases associated with degeneration of the eyeball, but is not limited thereto. In one embodiment, the eye disease includes, for example, keratoconus, cornea ectasia, Pellucid marginal corneal degeneration, corneal ulcer, infectious keratitis, bullous keratopathy, ulcerative keratitis, Ehlers-Danlos syndrome, myopia, posterior staphyloma, scleral ectasia, necrotizing scleritis, posterior scleritis, congenital glaucoma (buphthalmos), uveitis (complication), postoperative scarring such as minimally invasive glaucoma surgery, retinal detachment, and choroidal neovascularization. When crosslinking treatment is performed to the sclera of the back part of the eye ranging from the equatorial region of the eye to the posterior pole of the eye, the target eye disease is preferably myopia, posterior staphyloma, scleral ectasia, necrotizing scleritis, posterior scleritis, congenital glaucoma (buphthalmos), uveitis (complication), postoperative scarring such as minimally invasive glaucoma surgery, retinal detachment, or choroidal neovascularization, and more preferably myopia or posterior staphyloma. Myopia is categorized into axial myopia, refractive myopia, and pseudomyopia, and is particularly preferably axial myopia. Depending on the degree, myopia is also categorized into mild myopia, moderate myopia, excessive myopia, and pathological myopia, and is particularly preferably excessive myopia or pathological myopia. Myopia may also be associated with posterior staphyloma.

Light used for the crosslinking treatment as used herein may comprise light having a wavelength that is absorbable and excitable by a photoexcitable compound comprised in a photosensitizer used in the treatment, and is preferably light that does not significantly affect the safety of living bodies. For example, when the photoexcitable compound is excited by ultraviolet light, the light is preferably light comprising ultraviolet light. For example, when the photoexcitable compound is excited by blue light, the light is preferably light comprising blue light. For example, when the photoexcitable compound is riboflavin, the light is preferably light comprising ultraviolet light. The light for performing the crosslinking treatment is, for example, preferably light comprising light having a wavelength ranging from 300 to 550 nm, more preferably light comprising light having a wavelength ranging from 320 to 500 nm, still preferably light comprising light having a wavelength ranging from 320 to 460 nm, particularly preferably light comprising light having a wavelength ranging from 350 to 380 nm, and for example, it is light having a wavelength of 350 nm, 355 nm, 360 nm, 365 nm, 370 nm, 375 nm, or 380 nm.

The light "comprising light having a wavelength" as used herein may comprise a desired wavelength in the spectral distribution of the light. For example, the light "comprising light having a wavelength of 365 nm" may comprise a wavelength of 365 nm in the spectral distribution of the light. The light having a desired wavelength is preferably 10% or more, more preferably 20% or more, still preferably 30% or more, still further preferably 40% or more, and particularly preferably 50% or more, of the total output of light. The light having a desired wavelength may have the highest output among the spectral distribution of light. In one embodiment, light may be output from one or more light sources comprised in the light source device connected to the probe, and the light source device may be configured to vary the light intensity and wavelength of light to be output.

The illuminance of light for performing the crosslinking treatment as used herein may be any illuminance that does not significantly affect the safety of living bodies. When the illuminance of light is too high, the sclera irradiated by the light and tissues in the vicinity thereof are negatively affected. Whereas, when the illuminance is too low, the crosslinking treatment takes long and may cause insufficient effects. For example, it is preferably 1 mW/cm$^2$ or more, more preferably 1 to 30 mW/cm$^2$, still preferably 3 to 20 mW/cm$^2$, still further preferably 5 to 15 mW/cm$^2$, and particularly preferably 10 mW/cm$^2$.

Long-time irradiation of light for preforming the crosslinking treatment herein may put strains on patients or practitioners, whereas short-time irradiation may cause insufficient effects of the crosslinking treatment. For example, the irradiation time is preferably 30 seconds or more, more preferably 1 minute or more. Further, it is more preferably 1 to 10 minutes, still preferably 2 to 8 minutes, still further preferably 3 to 7 minutes, still further preferably 4 to 6 minutes, and particularly preferably 5 minutes. In another embodiment, the time may be 2 minutes, 3 minutes, 4 minutes, 6 minutes, 7 minutes, or 8 minutes. In one crosslinking treatment, light may be irradiated continuously for a predetermined duration, with a predetermined time interval, or with blinking. Preferably, light is irradiated continuously.

The crosslinking treatment herein is preferably peformed in the presence of the atmosphere, particularly in the presence of oxygen. The crosslinking treatment may provide insufficient effects because of insufficient amounts of oxygen required for the treatment when performed in living body sites where the treatment is carried out in close contact with living tissues. In order to deliver a sufficient amount of oxygen to living body sites, for example, putting space on a part of the probe may allow the probe to retain a sufficient amount of oxygen required for the crosslinking treatment, while the probe may be arranged in living body sites and used to peform the crosslinking treatment.

In one embodiment where the sclera of the posterior pole of the eye is subjected to crosslinking treatment, in order to arrange the probe accurately on the site in the posterior pole of the eye where the treatment is to be performed after arrangement of the probe on the posterior pole of the eye, for example, an accurate position of the probe in the posterior pole of the eye may be visually confirmed by outside visual observation of the inside part of the eye through emission of visible light from the probe by providing the probe with visible light from a light source device. The visible light provided to the probe is, for example, preferably light having a wavelength ranging from 500 to 800 nm, and the visible light with good visibility is, for example, preferably red light (600 to 800 nm) or green light (490 to 580 nm).

The light-emitting conditions and methods for the crosslinking treatment as used hererin may be optionally combined with each other.

In one embodiment, the photosensitizer may be administered before or after arrangement of the probe in a living body site. The time from administration of the photosensitizer to irradiation of light from the probe arranged at a living body site is preferably short, for example within 1 hour, preferably within 45 minutes, more preferably within 30 minutes, still preferably within 20 minutes, and particularly preferably within 15 minutes.

The photosensitizer, the probe, and the light source device as used herein may be provided as a kit comprising at least one of them. The kit may further comprise a cautionary note or a package insert indicating that the photosensitizer, the probe, and/or the light source device may be used or are to be used for prevention and/or treatment of eye diseases. The cautionary note or package insert may indicate an approval for manufacture, sale, and/or use of medicines and medical devices by the government or institution that regulates the manufacture, sale, and/or use.

The term "crosslinking of collagen fibers" as used herein refers to enhancement of the strength (stiffness) of collagen fibers in living body sites, which includes improvement of ratios of crosslinking of collagen fibers.

EXAMPLES

Formulation examples of photosensitizers and results of each test are illustrated as below, but these are intended to be described to understand the present invention in detail and are not intended to limit the scope of the present invention.

Formulation examples

Typical formulation examples of photosensitizers are shown as below. The contents of ingredients comprised in the following formulation examples are the content comprised in a 100-mL formulation.

Formulation example 1

| | |
|---|---|
| riboflavin sodium phosphate (flavin mononucleotide monosodium salt) | 0.1 g |
| citric acid hydrate | 0.5 g |
| sodium hydrogen phosphate dihydrate | 0.2 g |
| diluted hydrochloric acid | moderate amount |
| sodium hydroxide | moderate amount |
| purified water | moderate amount |
| pH | 7.0 |

Formulation example 2

| | |
|---|---|
| riboflavin sodium phosphate | 0.2 g |
| sodium hydrogen phosphate dihydrate | 0.15 g |
| sodium chloride | 0.9 g |
| diluted hydrochloric acid | moderate amount |
| sodium hydroxide | moderate amount |
| purified water | moderate amount |
| pH | 7.0 |

Formulation example 3

| | |
|---|---|
| flavin adenine dinucleotide sodium | 0.3 g |
| sodium citrate hydrate | 0.4 g |
| sodium chloride | 0.7 g |
| diluted hydrochloric acid | moderate amount |
| sodium hydroxide | moderate amount |
| purified water | moderate amount |
| pH | 6.5 |

Test examples

Test example 1. Effect of concentrations of a photosensitizer on the strength of sclera

(1) Preparation of photosensitizers

A predetermined amount of riboflavin-5'-phosphoric acid was dissolved in water so as to reach each concentration as shown in the following Table 1, followed by filter sterilization to prepare Formulations 1 to 4.

Table 1

| Concentration [%(w/v)] | Formulation 1 | Formulation 2 | Formulation 3 | Formulation 4 |
|---|---|---|---|---|
| riboflavin-5'- phosphoric acid | 0.002 | 0.02 | 0.2 | 0.5 |

(2) Provision of a probe

A probe comprising an emission body wherein the leading end part is discoidal (diameter of disc part: 6 mm, long-side length: 10 mm, thickness: 2 mm, made from acrylic resins) and a plate with 1-mm thickness (comprising 7 pores, each of which had diameter of a pore: 1.5 mm and depth of a pore: 0.7 mm, made from acrylic resins) was provided. The probe was connected to a light source device (ANUJ3500 manufactured by Panasonic).

(3) Test method

Procedure 1 Formulations 1 to 4 were administered in 350 µL under the capsule of Tenon of one eye of a rabbit under anesthesia.
Procedure 2 The capsule of Tenon was cut open 15 minutes after the administration, and a probe was inserted from the incision site along the sclera to be arranged at the posterior pole. A light source device was set up to irradiate ultraviolet light (wavelength: 365 nm, illuminance: 15 mW/cm$^2$) to the sclera of the posterior pole for 5 minutes.
Procedure 3 The eyeball was isolated, and then a piece of the sclera tissue with 10 mm of length and 5 mm of width was prepared. The piece of the sclera tissue was fixed on EZ Test (compact desktop testing machine, manufactured by Shimadzu, model name: EZ-SX) with a jig distance of 2 mm. The fixed tissue piece was put a preload of 0.1N for 1 minute on, followed by being pulled at a speed of 1 mm/min. The tension (N) in the case of 0.6 mm for displacement was adopted as an index of the strength of the sclera.
The sample which the above procedures 1 to 3 were conducted was referred to as a treated eye, whereas only the above procedure 3 was conducted for each corresponding contralateral eye as a comparison control. Eight examples for each formulation were performed, and the average value of the tension and the standard error were calculated according to conventionally used methods. The ratio of the strength was calculated based on the following equation.

$$\text{Ratio of strength} = \text{(Average value of treated eyes)}/\text{(Average value of contralateral eyes)}$$

(4) Test results and consideration

The test results are shown in Table 2. In any of Formulations 1 to 4, the strengths of the sclera in the treated eyes were enhanced compared to the contralateral eyes. In particular, when the concentration of the photoexcitable compound in a photosensitizer was 0.02%(w/v) or more, the strength of the sclera was sufficiently enhanced with a maximum at 0.2%(w/v).

Table 2

| Dosing formulation | Tension (N) in 0.6 mm of displacement | | Ratio of strength |
|---|---|---|---|
| | Treated eyes | Contralateral eyes | |
| Formulation 1 | 2.77 $\pm$ 0.34 | 2.01 $\pm$ 0.25 | 1.38 |
| Formulation 2 | 4.21 $\pm$ 0.42 | 2.46 $\pm$ 0.27 | 1.71 |
| Formulation 3 | 5.13 $\pm$ 0.38 | 1.69 $\pm$ 0.12 | 3.04 |
| Formulation 4 | 4.55 $\pm$ 0.25 | 1.86 $\pm$ 0.14 | 2.45 |

Test example 2. Effect of the illuminance of ultraviolet on the strength of sclera

(1) Preparation of a photosensitizer

A predetermined amount of riboflavin-5'-phosphoric acid was dissolved in water so as to reach the concentration of the following Table 3, followed by filter sterilization to prepare Formulation 5.

Table 3

| Concentration [%(w/v)] | Formulation 5 |
|---|---|
| riboflavin-5'-phosphoric acid | 0.25 |

(2) Provision of a probe

The same probe as the probe described in the afore-mentioned Test example 1 was provided. The probe was connected to a light source device (ANUJ3500 manufactured by Panasonic).

(3) Test method

Procedure 1 Formulation 5 was administered in 350 pL under the capsule of Tenon of one eye of a rabbit under anesthesia. Procedure 2 The capsule of Tenon was cut open 15 minutes after the administration, and a probe was inserted from the incision site along the sclera to be arranged at the posterior pole. A light source device was set up to irradiate ultraviolet light (wavelength: 365 nm) with illuminance of 1, 3, 10, or 30 $mW/cm^2$ to the sclera of the posterior pole for 5 minutes.

Procedure 3 The eyeball was isolated, and then a piece of the sclera tissue with 10 mm of length and 5 mm of width was prepared. The piece of the sclera tissue was fixed on EZ Test (compact desktop testing machine, manufactured by Shimadzu, model name: EZ-SX) with a jig distance of 2 mm. The fixed tissue piece was put a preload of 0.1N for 1 minute on, followed by being pulled at a speed of 1 mm/min. The tension (N) in the case of 0.6 mm for displacement was adopted as an index of the strength of the sclera.

The sample which the above procedures 1 to 3 were conducted was referred to as a treated eye, whereas only the above procedure 3 was conducted for each corresponding contralateral eye as a comparison control. Eight examples for each illuminance of the ultraviolet light were performed, and the average value of the tension and the standard error were calculated according to conventionally used methods. The ratio of the strength was calculated based on the following equation.

$$\text{Ratio of strength} = \text{(Average value of treated eyes)} / \text{(Average value of contralateral eyes)}$$

(4) Test results and consideration

The test results are shown in Table 4. In any of the illuminances of ultraviolet, the strengths of the sclera in the treated eyes were enhanced compared to the contralateral eyes. In particular, when the illuminance of ultraviolet was 3 $mW/cm^2$ or more, the strength of the sclera was sufficiently enhanced. It was shown that a stronger illuminance of ultraviolet enhanced the strength of the sclera.

Table 4

| Illuminance of ultraviolet [$mW/cm^2$] | Tension (N) in 0.6 mm of displacement | | Ratio of strength |
|---|---|---|---|
| | Treated eyes | Contralateral eyes | |
| 1 | 3.61 $\pm$ 0.39 | 2.67 $\pm$ 0.30 | 1.35 |
| 3 | 5.38 $\pm$ 0.73 | 2.99 $\pm$ 0.26 | 1.80 |
| 10 | 4.64 $\pm$ 0.39 | 2.56 $\pm$ 0.23 | 1.81 |
| 30 | 5.13 $\pm$ 0.60 | 2.33 $\pm$ 0.29 | 2.20 |

Test example 3. Effect of irradiation time of ultraviolet on the strength of sclera

(1) Preparation of a photosensitizer

Formulation 3 described in the afore-mentioned Test example 1 was used.

(2) Provision of a probe

The same probe as the probe described in the afore-mentioned Test example 1 was provided. The probe was connected to a light source device (ANUJ3500 manufactured by Panasonic).

(3) Test method

The test was performed according to the methods described in an academic literature "J Cataract Refact Surg., 2004, 30(3), 689-695" (Non Patent Literature 1).
Procedure 1 A piece of the sclera tissue of a rabbit with 10 mm of length and 5 mm of width was immersed in Formulation 3 for 15 minutes.
Procedure 2 The probe was arranged on the piece of the sclera tissue, and a light source device was set up to irradiate ultraviolet light (wavelength: 365 nm, illuminance: 10 mW/cm$^2$). The irradiation time was 1, 2, 3, 5, or 10 minutes.
Procedure 3 The piece of the sclera tissue was fixed on EZ Test (compact desktop testing machine, manufactured by Shimadzu, model name: EZ-SX) with a jig distance of 2 mm. The fixed tissue piece was put a preload of 0.1N for 1 minute on, followed by being pulled at a speed of 1 mm/min. The tension (N) in the case of 0.6 mm for displacement was adopted as an index of the strength of the sclera.
The sample which the above procedures 1 to 3 were conducted was referred to as a treated tissue piece, whereas the sample which only the above procedure 3 was conducted was referred to as a control tissue piece. Four examples for each irradiation time were performed, and the average value of the tension and the standard error were calculated according to conventionally used methods.

(4) Test results and consideration

The test results are shown in Table 5. In any of the irradiation times, the strengths of the sclera in the treated tissue pieces were enhanced compared to the control tissue piece. It was shown that a longer irradiation time enhanced the strength of the sclera.

Table 5

| Irradiation time [min] | Tension (N) in 0.6 mm of displacement |
|---|---|
| 0 (control tissue piece) | 3.78 ± 0.49 |
| 1 | 7.76 ± 0.54 |
| 2 | 9.02 ± 1.59 |
| 3 | 9.86 ± 0.92 |
| 5 | 8.71 ± 0.80 |
| 10 | 10.65 ± 1.34 |

Test example 4. Strength test of the sclera

(1) Preparation of a photosensitizer

Formulation 3 described in the afore-mentioned Test example 1 was used.

(2) Provision of a probe

The same probe as the probe described in the afore-mentioned Test example 1 was provided. The probe was connected to a light source device (ANUJ3500 manufactured by Panasonic).

(3) Test method

Procedure 1 Formulation 3 was administered in 230 µL under the capsule of Tenon of one eye of a rabbit under anesthesia. Procedure 2 The capsule of Tenon was cut open 15 minutes after the administration, and a probe was inserted from the incision site along the sclera to be arranged at the posterior pole. A light source device was set up to irradiate ultraviolet light (wavelength: 365 nm, illuminance: 10 mW/cm$^2$) to the sclera of the posterior pole for 5 minutes.

Procedure 3 The eyeball was isolated, and then a piece of the sclera tissue with 10 mm of length and 5 mm of width was prepared. The piece of the sclera tissue was fixed on EZ Test (compact desktop testing machine, manufactured by Shimadzu, model name: EZ-SX) with a jig distance of 2 mm. The fixed tissue piece was put a preload of 0.1N for 1 minute on, followed by being pulled at a speed of 1 mm/min. The tension (N) in the case of 0.6 mm for displacement was adopted as an index of the strength of the sclera.

The sample which the above procedures 1 to 3 were conducted was referred to as a treated eye, whereas only the above procedure 3 was conducted for each corresponding contralateral eye as a comparison control. Eight examples were performed, and the average value of the tension and the standard error were calculated according to conventionally used methods. The ratio of the strength was calculated based on the following equation.

$$\mathrm{Ratio\ of\ strength\ =\ (Average\ value\ of\ treated\ eyes)/(Average\ value\ of\ contralateral\ eyes)}$$

(4) Test results and consideration

The test results are shown in Table 6. The strength of the sclera in the treated eyes were enhanced compared to the contralateral eyes. In a histopathological test performed for the treated eyes 3 days after the crosslinking treatment, denaturation and necrosis of extraocular muscles associated with insertion of the probe were observed, but no effects on retina, choroid, or optic nerve were observed. The illuminance and irradiation time of ultraviolet light in this test were not considered to impact on visual performance involving these tissues.

Table 6

| Dosing formulation | Tension (N) in 0.6 mm of displacement | | Ratio of strength |
|---|---|---|---|
| | Treated eyes | Contralateral eyes | |
| Formulation 3 | 5.15 ± 0.83 | 1.79 ± 0.11 | 2.88 |

Test example 5. Effect of light wavelength on the strength of sclera

In order to assess the effect of light wavelength on the strength of sclera, the following test was conducted with a conventionally used probe.

(1) Preparation of a photosensitizer

Formulation 3 described in the afore-mentioned Test example 1 was used.

(2) Provision of a probe

A general medical device, illumination probe (20G, manufactured by FiberTech Co., Ltd.) was provided. The probe was connected to a light source device (3 LED light source device FL-301, manufactured by FiberTech Co., Ltd.).

(3) Test method

The test was performed according to the methods described in an academic literature "J Cataract Refact Surg., 2004, 30(3), 689-695" (Non Patent Literature 1).

Procedure 1 A piece of the sclera tissue of a rabbit with 10 mm of length and 5 mm of width was immersed in Formulation 3 for 15 minutes.
Procedure 2 The probe was arranged on the piece of the sclera tissue, and a light source device was set up to irradiate blue light (wavelength: 453 nm, illuminance: 50 mW/cm$^2$). The irradiation time was 1, 5, or 30 minutes. Procedure 3 The piece of the sclera tissue was fixed on EZ Test (compact desktop testing machine, manufactured by Shimadzu, model name: EZ-SX) with a jig distance of 2 mm. The fixed tissue piece was put a preload of 0.1N for 1 minute on, followed by being pulled at a speed of 1 mm/min. The tension (N) in the case of 0.8 mm for displacement was adopted as an index of the strength of the sclera.

The sample which the above procedures 1 to 3 were conducted was referred to as a treated tissue piece, whereas the sample which only the above procedure 3 was conducted was referred to as a control tissue piece. Three examples for each irradiation time were performed, and the average value of the tension and the standard error were calculated according to conventionally used methods.

(4) Test results and consideration

The test results are shown in Table 7. It was shown that in any of the irradiation times of even blue light, the strengths of the sclera in the treated tissue pieces were enhanced compared to the control tissue piece.

Table 7

| Irradiation time [min] | Tension (N) in 0.8 mm of displacement |
| --- | --- |
| 0 (control tissue piece) | 2.10 ± 0.36 |
| 1 | 10.82 ± 1.59 |
| 5 | 8.79 ± 2.31 |
| 30 | 7.83 ± 0.88 |

Test example 6. Crosslinking test of collagen fibers

For simplified assessment of degrees of crosslinking of collagen fibers by the crosslinking treatment, the following test was conducted with a conventionally used probe.

(1) Preparation of a photosensitizer

Formulation 5 described in the afore-mentioned Test example 2 was used.

(2) Test method

Procedure 1 A piece of collagen fiber sheet (Nippi. Inc., product code: 892201) of 5 mg by weight was used, and immersed in Formulation 5 for 3 minutes.
Procedure 2 The piece of collagen fiber sheet was irradiated ultraviolet light (wavelength: 365 nm, illuminance: 3 mW/cm$^2$) with a light for ultraviolet light irradiation (LED365-SPT/L, manufactured by Optocode Corporation) for 30 minutes. The piece of sheet which ultraviolet light was directly irradiated was referred to as Sample 1, and the piece of sheet which ultraviolet light was irradiated while putting a cover glass (0.12 to 0.17 mm thickness) on the piece so as not to be in contact with the atmosphere was referred to as Sample 2.
Procedure 3 The piece of collagen fiber sheet was immersed in 1-mL aqueous solution of 0.1% picrylsulfonic acid at 40°C for 2 hours. Then, 50 μL of the solution was taken out to be diluted 20 times with water for injection. Absorbance of the diluted solution at 345 nm was measured with an absorbance plate reader (SPECTRAmax PLUS 384, manufactured by Molecular Devices).

The above procedures 1 to 3 were conducted for Samples 1 and 2, whereas the piece of sheet which only the above procedure 3 was conducted was referred to as Sample 3 (control group). The percentages (%) of the collagen crosslinking of Samples 1 and 2 were calculated based on the following equation.

$$\text{Percentage (\%) of collagen crosslinking} = 100 \times ([\text{Absorbance of control group}] - [\text{Absorbance of example}])/([\text{Absorbance of control group}])$$

Two examples were conducted each for Samples 1 and 2, and the average value of the percentages of the collagen crosslinking of each group was calculated.

(3) Test results and consideration

The test results are shown in Table 8. In both of Samples 1 and 2, development of crosslinking of collagen fibers was observed.

Further, the crosslinking reaction of collagen fibers was observed to progress more in Sample 1 which ultraviolet light was irradiated in contact with the atmosphere than in Sample 2 which ultraviolet light was irradiated without contact with the atmosphere.

The result indicated that it is preferable to irradiate the sclera with ultraviolet light in contact with the atmosphere upon the crosslinking treatment of collagen fibers. Accordingly, it is considered that supply of the atmosphere may progress crosslinking of collagen fibers more efficiently in the vicinity of the posterior pole of eyeball which is no contact with the atmosphere.

Table 8

| Percentage [%] of collagen crosslinking | Sample 1 | Sample 2 |
|---|---|---|
| | 16.6% | 10.8% |

INDUSTRIAL APPLICABILITY

Enhancement of the strength of the sclera by crosslinking of collagen fibers via crosslinking treatment to the sclera of the equatorial region of the eye and/or the posterior pole of the eye such as the fundus of the eye allows for prevention and/or treatment of eye diseases.

**Claims**

1. A photosensitizer composition for use in prevention and/or treatment of myopia, comprising a photoexcitable compound, wherein the prevention and/or treatment comprises crosslinking treatment in the sclera of the equatorial region of the eye or the posterior pole of the eye.

2. The composition of claim 1, wherein the composition is used to be administered to the subconjunctiva or the capsule of Tenon of the eye.

3. The composition of claim 1 or 2, wherein the crosslinking treatment comprises emitting toward the sclera light comprising light having a wavelength absorbed by the photoexcitable compound.

4. The composition of any one of claims 1 to 3, wherein the light in the crosslinking treatment comprises light having a wavelength ranging from 300 to 550 nm.

5. The composition of any one of claims 1 to 4, wherein the crosslinking treatment comprises emitting toward the sclera light having an illuminance ranging from 1 to 30 mW/cm$^2$.

6. The composition of any one of claims 1 to 5, wherein the crosslinking treatment comprises emitting light toward the sclera for 1 to 10 minutes.

7. The composition of any one of claims 1 to 6, wherein the crosslinking treatment comprises emitting light in the presence of the atmosphere.

8. The composition of any one of claims 1 to 7, for use as an injection.

9. The composition of any one of claims 1 to 8, wherein the photoexcitable compound is comprised in a concentration ranging from 0.001 to 1%(w/v).

10. The composition of any one of claims 1 to 9, wherein the photoexcitable compound is riboflavin, flavin mononucleotide, flavin adenine dinucleotide, or a salt or a hydrate thereof.

11. The composition of any one of claims 1 to 10, wherein the myopia is at least one myopia selected from the group consisting of axial myopia, excessive myopia, and pathological myopia.

12. The composition of any one of claims 1 to 10, wherein the myopia is associated with posterior staphyloma.

13. A kit comprising the photosensitizer composition of any one of claims 1 to 12 and a probe for emitting light comprising light having a wavelength absorbed by the photoexcitable compound comprised in the photosensitizer composition.

14. The kit of claim 13, wherein the probe is used to be arranged along the sclera at the equatorial region of the eye or the posterior pole of the eye.

15. The kit of claim 13 or 14, wherein the probe comprises an emission surface for emitting light, to which a member comprising at least one pore is attached.

16. The kit of any one of claims 13 to 15, wherein the light comprises light having a wavelength ranging from 300 to 550 nm.

17. The kit of any one of claims 13 to 16, wherein the light is emitted with an illuminance ranging from 1 to 30 mW/cm$^2$.

18. The kit of any one of claims 13 to 17, wherein the probe is used to emit the light for 1 to 10 minutes.

19. The kit of any one of claims 13 to 18, wherein the photosensitizer composition is enclosed in a unit-dose container.

20. The kit of any one of claims 13 to 19, further comprising a light source device connectable to the probe.

21. A method for preventing and/or treating myopia, comprising the following steps:

    (1) administering a photosensitizer composition comprising a photoexcitable compound to the subconjunctiva or the capsule of Tenon of the eye, and
    (2) emitting toward the sclera light comprising light having a wavelength absorbed by the photoexcitable compound, thereby resulting in crosslinking of a collagen fiber contained in the sclera.

22. A method for crosslinking a collagen fiber, wherein a photosensitizer composition comprising a photoexcitable compound administered to the subconjunctiva or the capsule of Tenon of the eye is involved in crosslinking of a collagen fiber in the sclera of the equatorial region or the posterior pole of the eye under irradiation of light comprising light having a wavelength absorbed by the photoexcitable compound.

23. A method for emitting light, comprising emitting toward the sclera light comprising light having a wavelength ranging from 300 to 550 nm with an illuminance ranging from 1 to 30 mW/cm$^2$ for 1 to 10 minutes.

24. Use of a photoexcitable compound in the manufacture of a photosensitizer composition for use in prevention and/or treatment of myopia, wherein the prevention and/or treatment comprises crosslinking treatment of the sclera at the equatorial region of the eye or the posterior pole of the eye.

25. A photoexcitable compound for use in prevention and/or treatment of myopia, wherein the prevention and/or treatment comprises crosslinking treatment of the sclera at the equatorial region of the eye or the posterior pole of the eye.

# EP 4 186 524 A1

**INTERNATIONAL SEARCH REPORT**

| International application No. |
| --- |
| PCT/JP2021/027154 |

**A. CLASSIFICATION OF SUBJECT MATTER**

Int.Cl. A61K41/00(2020.01)i, A61F9/007(2006.01)i, A61K31/525(2006.01)i, A61K31/661(2006.01)i, A61K31/7084(2006.01)i, A61K41/17(2020.01)i, A61P27/10(2006.01)i

FI: A61K41/00, A61F9/007180, A61F9/007130C, A61K31/525, A61K31/661, A61K31/7084, A61P27/10, A61K41/17

According to International Patent Classification (IPC) or to both national classification and IPC

**B. FIELDS SEARCHED**

Minimum documentation searched (classification system followed by classification symbols)

Int.Cl. A61K41/00, A61F9/007, A61K31/525, A61K31/661, A61K31/7084, A61K41/17, A61P27/10

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2017-514564 A (UNIVERSITAT LEIPZIG) 08 June 2017 (2017-06-08), paragraphs [0001], [0184]-[0187], [0194], [0195], [0206], [0226]-[0240], example 1, fig. 4, 17, paragraphs [0006], [0219] | 1-25 |
| X | JP 2015-530202 A (UNIVERSITAT LEIPZIG) 15 October 2015 (2015-10-15), paragraphs [0001], [0058]-[0060], [0067], [0068], [0075]-[0079], [0082], [0092]-[0094], [0098]-[0108], example 1, fig. 3, 5, paragraphs [0088], [0089], fig. 6, paragraph [0095] | 1-25 |

☒ Further documents are listed in the continuation of Box C.  ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 20 August 2021 | 31 August 2021 |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2021/027154

C (Continuation). DOCUMENTS CONSIDERED TO BE RELEVANT

| Category* | Citation of document, with indication, where appropriate, of the relevant passages | Relevant to claim No. |
|---|---|---|
| X | KWOK, S. J. J. et al., Selective equatorial sclera crosslinking in the orbit using a metal-coated polymer waveguide, Invest. Ophthalmol. Vis. Sci., 2019, 60, pp. 2563-2570, abstract, p. 2564, left column, third paragraph, fig. 1, 3-5, p. 2565, right column, last paragraph to p. 2566, right column, third paragraph, p. 2567, right column, second paragraph to p. 2568, left column, first paragraph, fig. 6, 7, p. 2563, left column, the text, first paragraph to right column, second paragraph, p. 2566, left column, first paragraph | 1-25 |
| X | ISELI, H. P. et al., Scleral cross-linking by riboflavin and blue light application in young rabbits: damage threshold and eye growth inhibition, Graefes. Arch. Clin. Exp. Ophthalmol., 2016, 254, pp. 109-122, abstract, fig. 5, p. 110, right column, second paragraph to p. 111, right column, second paragraph, p. 116, right column | 1-25 |
| P, X | US 2021/0015664 A1 (HOLLADAY, J. T.) 21 January 2021 (2021-01-21), abstract, fig. 6, paragraphs [0022]-[0030], [0037], [0066]-[0068] | 1-25 |

Form PCT/ISA/210 (second sheet) (January 2015)

**INTERNATIONAL SEARCH REPORT**
Information on patent family members

International application No.

PCT/JP2021/027154

| | | |
|---|---|---|
| JP 2017-514564 A | 08 June 2017 | US 2017/0087017 A1<br>paragraphs [0001], [0282]-[0287],<br>[0315], [0316], [0327],<br>[0348]-[0363], example 1,<br>fig. 4, 17, paragraphs [0006], [0341]<br>WO 2015/155117 A1<br>KR 10-2017-0002425 A<br>CN 106413644 A |
| JP 2015-530202 A | 15 October 2015 | US 2015/0257928 A1<br>paragraphs [0001], [0067]-[0073],<br>[0091], [0092], [0099]-[0101],<br>[0106], [0116]-[0123],<br>[0126]-[0137], example 1, fig. 3, 5,<br>paragraphs [0112], [0113], fig. 6<br>WO 2014/056895 A1<br>KR 10-2015-0095628 A<br>CN 104936563 A |
| US 2021/0015664 A1 | 21 January 2021 | (Family: none) |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *J Cataract Refract Surg,* 2004, vol. 30, 689-695
- *Acta Ophthalmol.,* 2009, vol. 87, 193-198
- *J Cataract Refract Surg,* 2013, vol. 39, 1184-1189
- *J Cataract Refact Surg.,* 2004, vol. 30 (3), 689-695